Europäisches Patentamt

European Patent Office

Office européen des brevets

(19)

(11) Veröffentlichungsnummer : **0 432 638 B1**

(12) # EUROPÄISCHE PATENTSCHRIFT

(45) Veröffentlichungstag der Patentschrift :
**15.09.93 Patentblatt 93/37**

(51) Int. Cl.$^5$ : **A61K 31/71**

(21) Anmeldenummer : **90123404.7**

(22) Anmeldetag : **06.12.90**

(54) **Kombinationspräparate enthaltend Chloramphenicol, Gentamicin und Nystatin als aktive Wirkstoffe zur topischen Behandlung entzündlicher Hauterkrankungen.**

Die Akte enthält technische Angaben, die nach dem Eingang der Anmeldung eingereicht wurden und die nicht in dieser Patentschrift enthalten sind.

(30) Priorität : **14.12.89 DE 3941263**

(43) Veröffentlichungstag der Anmeldung :
**19.06.91 Patentblatt 91/25**

(45) Bekanntmachung des Hinweises auf die Patenterteilung :
**15.09.93 Patentblatt 93/37**

(84) Benannte Vertragsstaaten :
**AT BE CH DE DK ES FR GB GR IT LI LU NL SE**

(56) Entgegenhaltungen :
**UNLISTED DRUGS, Band 35, Nr. 2, Februar 1983, Seite 23d, Chatham, NJ, US
UNLISTED DRUGS, Band 22, Nr. 8, August 1970, Seite 118b, Chatham, NJ, US; Seite 118b: "Cavumycina"
UNLISTED DRUGS, Band 18, Nr. 7, Juli 1966, Seite 65b, Chatham, NJ, US**

(73) Patentinhaber : **Abdulla, Souhail, Dr. med.
Rhauderwieke 1
D-26817 Rhauderfehn (DE)**

(72) Erfinder : **Abdulla, Souhail, Dr. med.
Rhauderwieke 1
D-26817 Rhauderfehn (DE)**

(74) Vertreter : **UEXKÜLL & STOLBERG
Patentanwälte
Beselerstrasse 4
D-22607 Hamburg (DE)**

EP 0 432 638 B1

Jouve, 18, rue Saint-Denis, 75001 PARIS

## Beschreibung

Die Erfindung betrifft Kombinationspräparate zur topischen Behandlung von entzündlichen Hauterkrankungen, und insbesondere von schwer heilenden Hauterkrankungen wie chronischen Ekzemen, ekzematösen Entzündungen im Anal- und Genitalbereich sowie von sekundärheilenden Wunden und Abszessen wie Ulcus cruris, Dekubitus und Furunkeln.

Das erfindungsgemäße Präparat kann ferner zur topischen Behandlung von Hautverbrennungen, Intertrigo und Impetigo sowie von chronisch unspezifischen Wunden im Mundwinkelbereich eingesetzt werden.

Die oben genannten Hauterkrankungen und sekundären Entzündungen wurden bereits bisher mit Antibiotika der verschiedensten Typen sowie mit Cortisonen behandelt. In vielen Fällen ist jedoch eine vollständige und dauerhafte Schließung der Wunden mit herkömmlichen Mitteln nicht zu erreichen. Dies gilt insbesondere für die verschiedenen Erscheinungsformen des Ulcus cruris sowie bei proktologischen Beschwerden wie Ekzemen und Fissuren im Analbereich. Derartige Wunden heilen erfahrungsgemäß sehr schlecht ab, und die nur oberflächig geschlossene Haut reißt bei Belastung schnell wieder auf. So können beispielsweise Analfissuren in schweren Fällen nur chirurgisch oder durch Verschorfen beseitigt werden. Eine derartige Behandlung kann jedoch zu schwerwiegenden Spätfolgen wie Stenosen führen.

Erfindungsgemäß hat es sich überraschenderweise gezeigt, daß durch Kombination der an sich bekannten Antibiotika Chloramphenicol (I-Chla), Gentamicin (II-Gt) und Nystatin (III-Ns) ein Präparat für die topische Applikation erhalten wird, welches selbst extreme und hartnäckige Hauterkrankungen und Sekundärwunden in verhältnismäßig kurzer Zeit dauerhaft heilt. So wurde insbesondere bei der Behandlung von Analfissuren in fortgeschrittenem Stadium in mehr als 80 % der Fälle eine vollständige Heilung erzielt.

Gemäß einer bevorzugten Ausführungsform der Erfindung enthält das Präparat zusätzlich Cortison (IV-Cort). Durch das Hinzutreten von Cortison gemäß der nachfolgenden Definition und in den unten angegebenen sehr niedrigen Mengen wird eine weitere Beschleunigung und Verstärkung des Therapieeffektes auf synergistischer Basis mit den übrigen Bestandteilen des Präparats nach der Erfindung erreicht (vgl. Beispiel 7).

Erfindungsgemäß schließen die für die oben genannten Wirkstoffe gewählten Bezeichnungen deren gleichwirkende Strukturanaloga und Derivate ein. Im Falle des Chloramphenicols kann es sich beispielsweise um dessen Stereoisomere sowie die Chloramphenicol-Panthotenat-Komplexe handeln. Im Falle des Gentamicins sind erfindungsgemäß Gentamicin $C_1$, $C_2$ und $C_{1A}$ sowie die Sulfate dieser Gentamicine eingeschlossen. Entsprechendes gilt für die verschiedenen Nystatine, wie beispielsweise Nystatin $A_1$ und $A_2$ und für die Cortisonderivate, wobei der Begriff "Cortison" erfindungsgemäß insbesondere Hydrocortison einschließt.

Die erfindungsgemäßen Kombinationspräparate können die aktiven Wirkstoffe I-Chla, II-Gt und III-Ns im Gewichtsverhältnis von (4 bis 12) zu (0,5 bis 1,5) zu (20 bis 60), vorzugsweise von (6 bis 10) zu (0,75 bis 1,25) zu (30 bis 50) und in besonders bevorzugter Weise von (8) zu (1) zu (40), jeweils bezogen auf das Gewicht der Basisverbindung enthalten.

Ist zusätzlich IV-Cort vorhanden, so beträgt das Gewichtsverhältnis der aktiven Wirkstoffe I-Chla, II-Gt, III-Ns und IV-Cort beispielsweise (4 bis 12) zu (0,5 bis 1,5) zu (20 bis 60) zu (1,5 bis 4,5), vorzugsweise (6 bis 10) zu (0,75 bis 1,25) zu (30 bis 50) zu (2 bis 4) und in besonders bevorzugter Weise (8) zu (1) zu (40) zu (3), jeweils bezogen auf das Gewicht der Basisverbindung.

Gemäß einer besonders bevorzugten Ausführung der Erfindung liegen die Kombinationspräparate in Salbenform vor. Zu diesem Zweck können die Wirkstoffe in übliche Salbengrundlagen eingearbeitet werden, wobei letztere unter Berücksichtigung der jeweils vorgesehenen Indikation ausgewählt werden sollten.

In Betracht kommen beispielsweise lipophile Grundlagen aus Kohlenwasserstoffen oder Glyceridfetten, wie Vaselin oder Öle oder Mischungen derselben, welche die eingearbeiteten Wirkstoffe langsam freisetzen und auf diese Weise Depotwirkung ausüben können. Besonders geeignet ist eine im Handel unter der Bezeichnung Plastibase erhältliche Zubereitung aus flüssigem Paraffin und Polyethylen.

Ferner können emulgierende Fettgrundlagen (Absorptionsgrundlagen) verwendet werden, die neben den genannten Fettstoffen (Vaselin, Paraffin, Glyceridfetten, Wachsen) noch Emulgatoren enthalten. Sie nehmen Wasser und wäßrige Lösungen unter Ausbildung stabiler Wasser-in-Öl-Emulsionen auf und eignen sich zur Herstellung wasserfreier sowie wasserhaltiger Salben, aus denen die Wirkstoffkombination zügig abgegeben wird. Geeignete Emulsionen lassen sich mit Emulgatormischungen herstellen, wie sie beispielsweise im Wollwachs vorliegen.

Gute Absorptionsgrundlagen für die erfindungsgemäßen Wirkstoffkombinationen sind beispielsweise Gemische aus Paraffin, Vaselin, einem Fettalkohol wie Cetylalkohol und Wollwachs oder Emulsionen aus Lazeran (Wollwachsalkoholsalbe nach DAB9-BRD), dickflüssigem Paraffin und Sorbol M (entsprechend Parben M) sowie Sorbol P (entsprechend Parben P). Anstelle von Wollwachs bzw. Wollwachsalkoholen und Derivaten können auch andere Emulgatoren und Emulgatorgemische wie z.B. Mono- und Diglyceride der Fettsäuren oder Ester bzw. Ether verschiedener Zucker mit Fettsäuren bzw. Fettalkoholen verwendet werden. So hat sich beispielsweise eine W/O-Emulsion aus Triglyceriden mittlerer Kettenlänge, dickflüssigem Paraffin und weißer Vaselin mit den Emulgatoren Propylenglykolmonostearat, Glycerinmonostearat, Cetylstearylalkohol, Propylenglykol und Polyethylenglykol-Sorbitan-Derivaten, gereinigtem Wasser und Siliciumdioxid in hochdisperser Form als geeignet erwiesen.

Für die Herstellung einer augenverträglichen Salbe kann eine der genannten Salbengrundlagen, jedoch ohne Siliciumdioxid, verwendet werden.

Erfindungsgemäß hat sich eine neutrale Salbengrundlage in Kombination mit mindestens etwa 20 bis 30 Gew.%, bezogen auf die Gesamtmischung, an Salbengrundlage PL (Plastibase) besonders bewährt.

Derartige Salben können erfindungsgemäß je 100 g Gesamtprodukt 100 bis 300 mg I-Chla, 10 bis 35 mg II-Gt und 700 bis 1100 mg III-Ns und gegebenenfalls 60 bis 100 mg IV-Cort enthalten.

Gemäß einer besonders bevorzugten Ausführungsform der Erfindung enthält eine derartige Salbe etwa 200 mg I-Chla, etwa 25 mg II-Gt, etwa 900 mg III-Ns und etwa 80 mg IV-Cort je 100 g Gesamtprodukt.

Die erfindungsgemäßen Kombinationspräparate können ferner als Lotionen sowie in augenverträgliche Flüssigkeiten zum Tropfen in einer Konzentration von 0,8 bis 1,5 Gew.% unter Beibehaltung der oben ausgeführten Mengenverhältnisse der Wirkstoffe zueinander eingearbeitet werden. Als Flüssigkeitsbasis kommen beispielsweise pflanzliche Öle, wie Oleum neutralium, sowie destilliertes Wasser in Betracht.

Überraschenderweise hat es sich erfindungsgemäß gezeigt, daß mit Präparaten, die die oben genannten Wirkstoffe in Kombination enthalten, ein therapeutischer Effekt eintritt, wie er durch die Einzelkomponenten oder bekannte Kombinationen derselben, wie Chloramphenicol/Cortison, Gentamicin/Cortison und Nystatin/Cortison in therapeutisch vertretbaren Dosierungen nicht erreicht werden kann. In diesem Zusammenhang liegt ein besonderer Vorteil der erfindungsgemäßen Präparate in dem Umstand, daß die bezüglich ihrer Nebenwirkungen problematische Gentamicinkomponente sowie gegebenenfalls das Cortison in Dosierungen weit unterhalb der kritischen Grenze vorhanden ist, andererseits jedoch durch das Zusammenwirken der Bestandteile ein überraschender therapeutischer Effekt erzielt wird. Es handelt sich demgemäß um eine echte synergistische Wirksamkeit bei der Behandlung von ansonsten schwer behandelbaren, entzündlichen Hauterkrankungen. Überraschenderweise wurden Resistenzen auch bei Langzeitbehandlung nicht beobachtet.

Die Erfindung wird nachfolgend anhand von Beispielen erläutert.

## Beispiel 1

### Herstellung eines Salbenpräparats

Aus den folgenden Bestandteilen wurde eine Salbe hergestellt:

| | |
|---|---|
| Chloramphenicol | 200 mg |
| Gentamicinsulfat | 25 mg |
| Nustatin | 900 mg |
| Hydrocortison | 80 mg |

Salbengrundlage: Mischung aus Polyethylen und dünnflüssigem Paraffin (Plastibase) (50 g) und Lazeran (ad 100 g).

Die Bestandteile wurden auf übliche Weise homogen zu einer Salbe vermischt.

## Beispiel 2

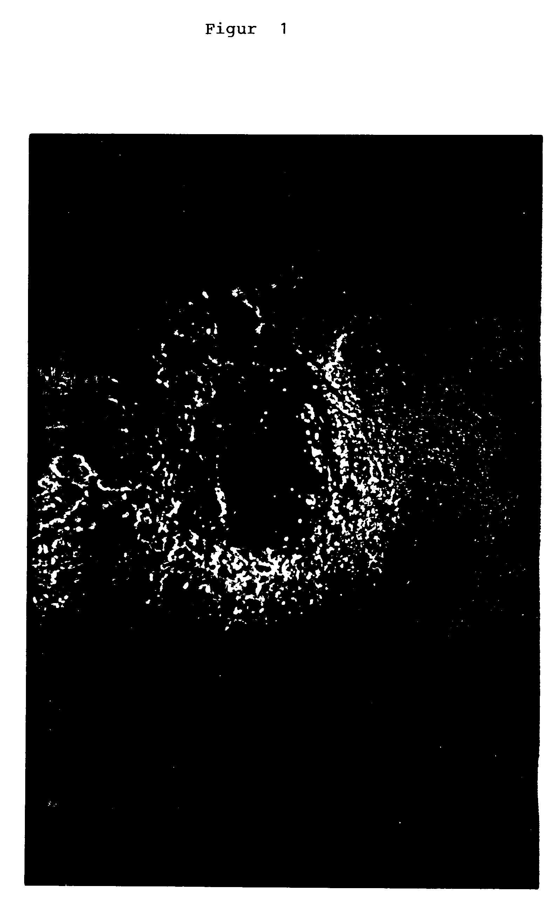

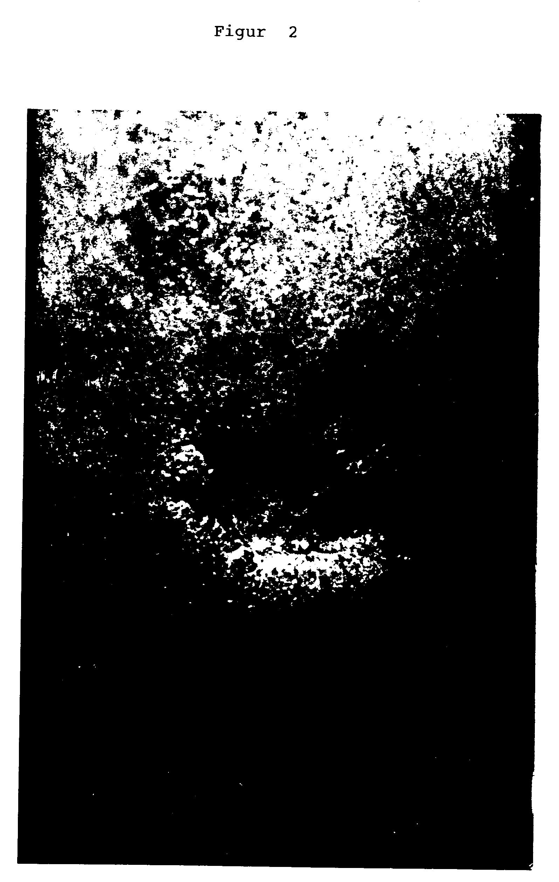

Eine etwa 50 Jahre alte Frau mit linksseitigem Ulcus cruris als Folge eines postthrombotischen Syndroms war etwa 1½ Jahre lang vergeblich mit herkömmlichen Mitteln behandelt worden. Das Geschwür wies bei einer Tiefe von 0,5 cm eine Größe von ungefähr 2 x 3 cm (vgl. Fig. 1) auf.

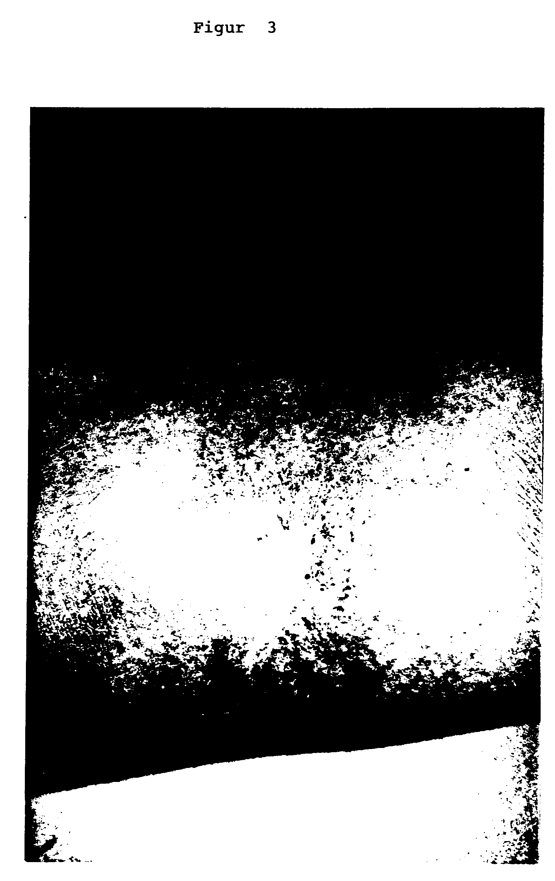

Nach Applikation der erfindungsgemäßen Salbe (vgl. Beispiel 1) dreimal täglich auf die offene Stelle trat bereits nach einer Behandlungswoche eine deutliche Besserung ein (vgl. Fig. 2).

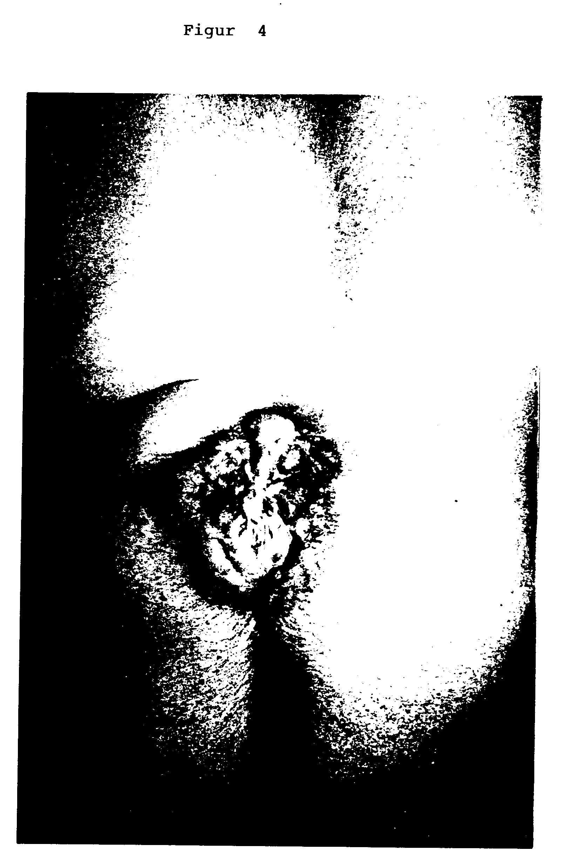

Nach weiteren 3½ Wochen, während derer die erfindungsgemäße Salbe zunächst dreimal und anschließend zweimal täglich aufgetragen wurde, trat eine vollständige Heilung ein. In dem ursprünglichen Wundbereich bildete sich eine stabile Hautschicht, die auch Belastungen, wie Stößen, gewachsen war (vgl. Fig. 3).

## Beispiel 3

Ein inzwischen etwa 12 Jahre alter Junge, der auf einen Geh-apparat angewiesen war, litt an offenen Druck- und Schürfstellen, die durch den Apparat hervorgerufen waren. Mit herkömmlichen Salben einschließlich Antibiotika oder Cortison enthaltenden Präparaten gelang es nicht, die sich immer wieder entzündenden Stellen dauerhaft zu heilen.

Nach Applikation der erfindungsgemäßen Salbe (vgl. Beispiel 1) schlossen sich die Wunden innerhalb von etwa 4 bis 7 Tagen. Durch prophylaktische Behandlung der gefährdeten Stellen 2- bis 3mal wöchentlich konnte das Auftreten erneuter Wundstellen dauerhaft vermieden werden. Die prophylaktische Behandlung wurde über einen Zeitraum von 8 Jahren durchgeführt. Während dieser Zeit trat keine Resistenz auf!

**Beispiel 4**

Der gleiche Junge wie in Beispiel 3 beschrieben wurde mit Spina bifida mit handflächengroßer thoracolumbaler Meningomyelozele geboren. Wegen der Größe der Cele kam eine Operation nicht in Betracht. Nach etwa 3 Monaten platzte die Cele und sonderte eine eitrige Flüssigkeit ab; wider Erwarten überlebte das Kind die Krise. Die Wunde wurde zunächst weitgehend erfolglos mit konventionellen Salben, einschließlich Antibiotika und Cortison enthaltenden Präparaten behandelt.

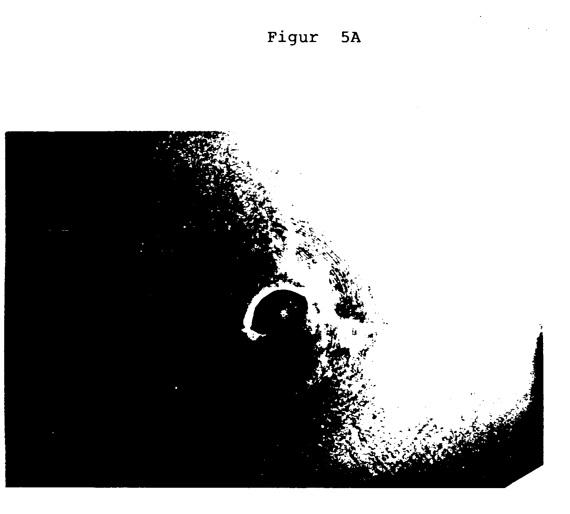

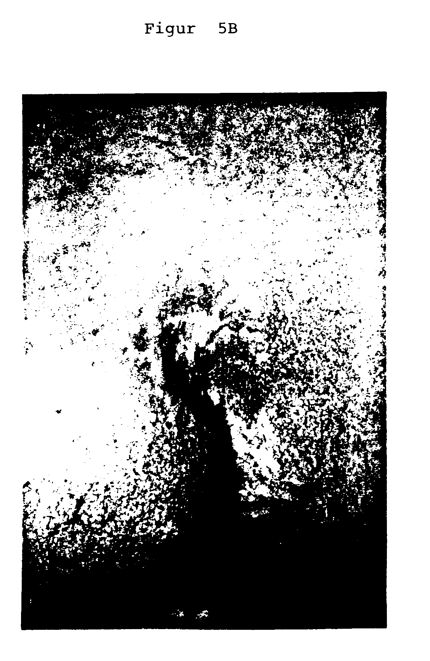

Sodann wurde das erfindungsgemäße Kombinationspräparat (vgl. Beispiel 1) eingesetzt, und die Wunde begann sich nach einigen Tagen zu schließen. Nach 3 bis 4 Wochen war die Wunde vollständig mit einer feinen Haut bedeckt, welche sich in den nachfolgenden Wochen zunehmend kräftigte. Die Wunde ist seither geschlossen (vgl. Figur 4).

**Beispiel 5**

Ein 33 Jahre alter Mann wurde in Israel bei einem Brandanschlag im Februar 1989 schwer verletzt und lag 3 Monate lang bewußtlos im Krankenhaus.

Als Folge der Bettlägerigkeit bekam er 3 Dekubituswunden, von denen eine etwa pflaumengroße offene Wunde sich am Steiß befand; eine zweite Wunde mit einem Durchmesser von etwa 8 x 10 cm lag im Bereich der rechten Hüfte, und eine dritte Wunde mit einem Durchmesser von etwa 7 x 9 cm befand sich an der linken Hüfte.

Die Tiefe der Wunden reichte bis zum Muskel. Die Wunden wurden zunächst in einem israelischen Krankenhaus mit Erythromycin-, Refobacin- und Leukomycinsalbe behandelt. Die Behandlung blieb jedoch erfolglos.

Der behandelnde Arzt versuchte sodann, die Wunden mit einer Kombination aus Leukomycin und Cortison zu therapieren.

Anschließend wurde in dem gleichen Krankenhaus versucht, die Wunden mit einer Kombination aus Refobacin und Cortison zu schließen. Auch dieser Behandlung blieb jedoch nach mehrwöchiger Dauer der Erfolg versagt.

Schließlich wurde der Versuch unternommen, die Wunden mit Hausmitteln wie Zucker- und Kochsalzlösungen, Desinfektionsmitteln und sogar mit Honig zu behandeln.

Der Patient wurde im Juni 1989 nach Deutschland überführt und lag dort etwa 3 Monate lang im Krankenhaus. Er hatte das Bewußtsein wiedererlangt, die Dekubituswunden heilten jedoch nicht.

Es wurde ein Facharzt für plastische Chirurgie hinzugezogen und dieser empfahl, die Wunden chiroplastisch zu versorgen. Er räumte jedoch nur eine Erfolgschance von etwa 50% ein. Die Dauer der Behandlung wurde auf 6 Monate und die Kosten auf etwa DM 100.000,- bis 150.000.- veranschlagt.

Die Durchführung dieser Behandlung war aus Kostengründen nicht möglich.

Der Patient wurde anschließend als Pflegefall bei seinem Bruder aufgenommen.

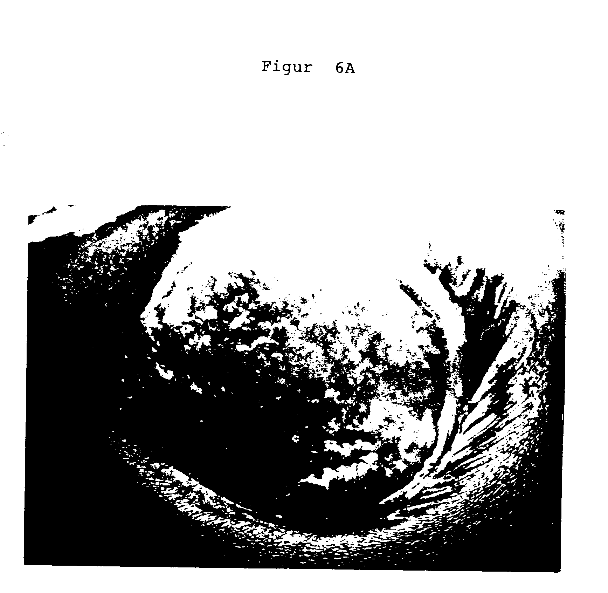

Als der Erfinder von diesem Fall hörte, hat er seine Hilfe angeboten. Er begann die Behandlung mit der erfindungsgemäßen Salbe (vgl. Beispiel 1) im Oktober 1989. Die Wunde am Steißbereich war bereits nach 5 Wochen reizlos geschlossen. Figur 5A ist eine photographische Abbildung der Wunde vor der Behandlung und Figur 5B nach 5- wöchiger Behandlung.

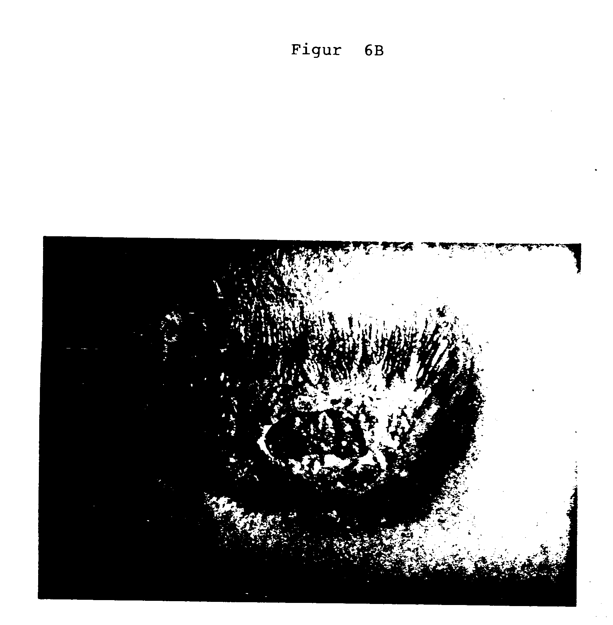

Die zweite Wunde an der Hüfte rechts war nach 11 Wochen bis auf einen kleinen Rest nahezu vollständig geschlossen. Figur 6A ist eine photographische Abbildung der Wunde vor der Behandlung und Figur 6B nach 11 wöchiger Behandlung.

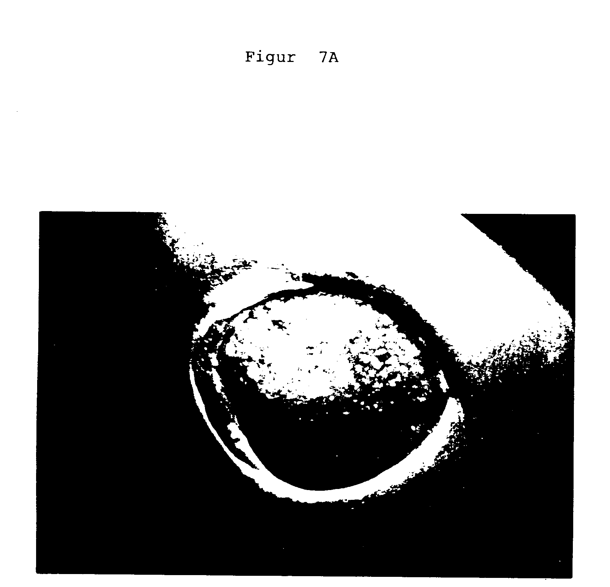

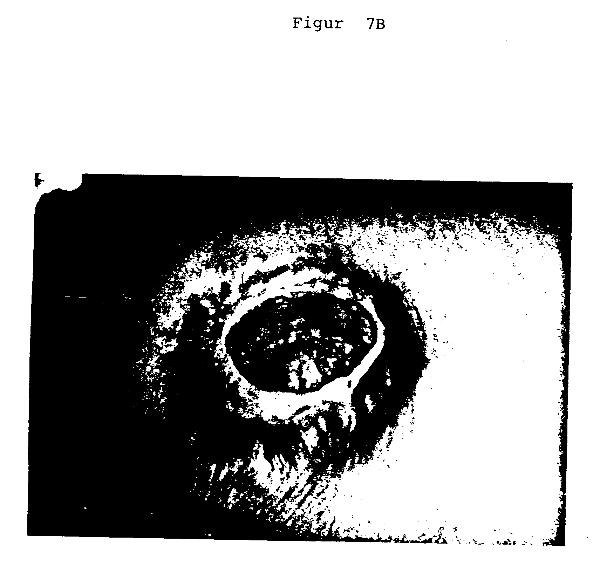

Die dritte Wunde an der linken Hüfte war nach 11 Wochen zu etwa 80% reizlos geschlossen. Figur 7A ist eine photographische Abbildung der Wunde vor der Behandlung und Figur 7B nach 11 wöchiger Behandlung.

In diesem Zustand wurde der Patient zu Anfang des Jahres 1990 zurück nach Israel geflogen. Die Behandlung mit der erfindungsgemäßen Salbe wurde dort fortgesetzt.

Kurze Zeit später waren alle Wunden vollständig verschlossen.

**Beispiel 6**

**Therapiebericht über 237 Patienten mit Entzündungen im Analbereich**

**A. Patientenstruktur:**

Die Gesamtzahl der Patienten betrug 237. Von diesen waren 112 weiblich und 125 männlich. Das Alter lag zwischen 18 und 69 Jahren, das Durchschnittsalter bei etwa 39 Jahren.

155 der Patienten litten an chronischen Analekzemen, 37 an chronischen Analfissuren und 45 an chronischen Analekzemen in Kombination mit Analfissuren.

Alle Patienten waren zuvor mehrmals mit verschiedenen der üblich topisch anwendbaren proktologischen Präparate erfolglos behandelt worden. Beispiele für die bereits eingesetzten Präparate sind Antibiotika, Antimycotica, Corticoidpräparate und physikalische Maßnahmen wie Sitzbäder.

169 der Patienten waren 1 bis mehrmals mit Kombinationspräparaten wie Chloramphenicol/Cortison, Gentamicin/Cortison oder Nystatin/Cortison ohne klinischen Erfolg behandelt worden.

**B. Therapievorschrift:**

Alle Patienten wendeten die erfindungsgemäße Salbe zweimal täglich an. Patienten mit Analfissuren wendeten die erfindungsgemäße Salbe (vgl. Beispiel 1) zusätzlich nach jedem Stuhlgang an

**C. Ergebnisse:**

1) Bei den Patienten mit Analekzemen verschwand der Juckreiz nach 2 bis 10 Tagen.
2) Bei den Patienten mit Analfissuren verschwanden die Defäkationsbeschwerden nach 3 bis nach 14 Tagen.

Die Patienten galten als geheilt, wenn sie mindestens 10 Wochen nach Beendigung der Salbenanwendung beschwerdefrei geblieben waren. Sie wurden im Rhythmus von 1 bis 2 Wochen kontrolliert. Im Durchschnitt trat der vollständige Heilerfolg nach etwa 14 Tagen auf.

Die Erfolgsrate ist nachfolgend in Tabelle 1 wiedergegeben:

**Tabelle 1**

|  | Pat.-Zahl | Geheilt | Gebessert |
|---|---|---|---|
| Chronische Analekzeme | 155 = 100% | 138 = 89% | 17 = 11% |
| Chronische Analfissuren | 37 = 100% | 31 = 84% | 4 = 16% |
| Chronische Analekzeme mit Analfissuren | 45 = 100% | 36 = 80% | 9 = 20% |

Die Ergebnisse zeigen, daß das erfindungsgemäße Kombinationspräparat bei der Behandlung von Analekzemen und Analfissuren oder Kombinationen derselben überraschenderweise eine ausgezeichnete Wirkung mit einer Heilungsrate von 80 bis 89% entfaltet.

In diesem Zusammenhang ist hervorzuheben, daß die einzelnen Wirkstoffe jeweils in sehr niedriger Dosierung vorliegen, jedoch in Kombination eine extrem hohe und bisher nicht beobachtete Wirksamkeit zeigen. Diese Wirkung ist nach den klinischen Beobachtungen nur durch eine synergistische Wirkung erklärbar.

**D. Nebenwirkungen:**

Lediglich bei 33 der behandelten Patienten (15,6%) trat zu Beginn der Behandlung nach Anwendung der Salbe ein etwa 5 bis 15 Minuten lang andauerndes leichtes Brennen auf. Das Brennen hörte jedoch nach wenigen Tagen der Anwendung auf. Sonstige Nebenwirkungen oder Allergien wurden nicht beobachtet.

**Beispiel 7**

Eine parallele Gruppe von 20 Patienten, die an chronischen Analekzemen litten, wurden wie in Beispiel 6 angegeben behandelt, mit dem Unterschied, daß die erfindungsgemäße Salbe ohne die Cortisonkomponente verwendet wurde.

Die Patientenstruktur entsprach ungefähr der Struktur wie in Beispiel 6 angegeben, d.h. das Durchschnittsalter betrug etwa 40 Jahre.

Der therapeutische Erfolg wie in Beispiel 6 beschrieben wurde im Prinzip auch mit dem Cortison-freien Präparat erreicht, es zeigte sich lediglich, daß das Verschwinden des Juckreizes im Durchschnitt 1 bis 2 Tage und die vollständige Heilung 2 bis 4 Tage später eintrat als in Beispiel 6 beschrieben.

Die Ergebnisse dieser Untersuchungsserie zeigen, daß der überraschende therapeutische Effekt durch das synergistische Zusammenwirken der Komponenten Chloramphenicol, Gentaicin und Nystatin zustande kommt, während das Cortison den therapeutischen Effekt im Zusammenwirken mit den übrigen Bestandteilen bereits in außerordentlich niedrigen Mengen verstärkt bzw. beschleunigt.

**Patentansprüche**

1. Kombinationspräparat zur topischen Behandlung entzündlicher Hauterkrankungen enthaltend Chloramphenicol (I-Chla), Gentamicin (II-Gt) und Nystatin (III-Ns) als aktive Wirkstoffe.

2. Kombinationspräparat nach Anspruch 1, **dadurch gekennzeichnet**, daß es die Wirkstoffe I-Chla, II-Gt und III-Ns im Verhältnis (4 bis 12) zu (0,5 bis 1,5) zu (20 bis 60) enthält.

3. Kombinationspräparat nach den Ansprüchen 1 oder 2, dadurch gekennzeichnet, daß es zusätzlich Cortison (IV-Cort) enthält.

4. Kombinationspräparat nach Anspruch 3, **dadurch gekennzeichnet**, daß es die Wirkstoffe I-Chla, II-Gt, III-Ns und IV-Cort im Verhältnis (4 bis 12) zu (0,5 bis 1,5) zu (20 bis 60) zu (1,5 bis 4,5) enthält.

5. Kombinationspräparat nach den Ansprüchen 1 oder 2, **dadurch gekennzeichnet**, daß es die Wirkstoffe als Mischung in einer Salbengrundlage enthält.

6. Kombinationspräparat nach Anspruch 5, **dadurch gekennzeichnet**, daß es je 100 g Salbenmischung 100 bis 300 mg I-Chla, 10 bis 35 mg II-Gt und 700 bis 1100 mg III-Ns enthält.

7. Kombinationspräparat nach den Ansprüchen 3 oder 4, **dadurch gekennzeichnet**, daß es die Wirkstoffe als Mischung in einer Salbengrundlage enthält.

8. Kombinationspräparat nach Anspruch 3, **dadurch gekennzeichnet,** daß es je 100 g Salbenmischung 100 bis 300 mg I-Chla, 10 bis 35 mg II-Gt, 700 bis 1100 mg III-Ns und 60 bis 100 mg IV-Cort enthält.

9. Kombinationspräparat nach den Ansprüchen 1 bis 4, **dadurch gekennzeichnet,** daß es die Wirkstoffe in einer augenverträglichen Flüssigkeit enthält.

**Claims**

1. Combination preparation for the topical treatment of inflammatory skin diseases, comprising chloramphenicol (I-Chla), gentamicin (II-Gt) and nystatin (III-Ns) as active ingredients.

2. Combination preparation according to claim 1, characterized in that it comprises the ingredients I-Chla, II-Gt and III-Ns in the ratio (4 to 12) to (0.5 to 1.5) to (20 to 60).

3. Combination preparation according to claims 1 or 2, characterized in that it additional comprises cortisone (IV-Cort).

4. Combination preparation according to claim 3, characterized in that it contains the ingredients I-Chla, II-Gt, III-Ns and IV-Cort in the ratio (4 to 22) to (0.5 to 1.5) to (20 to 60) to (1.5 to 4.5).

5. Combination preparation according to claims 1 or 2, characterized in that it comprises the ingredients as a mixture in an ointment base.

6. Combination preparation according to claim 5, characterized in that it comprises 100 to 300 mg I-Chla, 10 to 35 mg II-Gt and 700 to 1100 mg III-Ns per 100 g ointment mixture.

7. Combination preparation according to claims 3 or 4, characterized in that it comprises the ingredients as a mixture in an ointment base.

8. Combination preparation according to claim 3, characterized in that it comprises 100 to 300 mg I-Chla, 10 to 35 mg II-Gt, 700 to 1100 mg III-Ns and 60 to 100 mg IV-Cort per 100 g ointment mixture.

9. Combination preparation according to claims 1 to 4, characterized in that it contains the ingredients in a fluid acceptable for administration to the eyes.

**Revendications**

1. Préparation en association pour le traitement topique des maladies d'inflammation de la peau contenant du chloramphénicol (I-Chla), de la gentamicine (II-Gt) et de la nystatine (III-Ns) en tant qu'agents actifs.

2. Préparation en association selon la revendication 1, caractérisée en ce qu'elle contient les agents actifs I-Chla, II-Gt et III-Ns aux proportions de (4 à 12) à (0,5 à 1,5) à (20 à 60).

3. Préparation en association selon les revendications 1 ou 2, caractérisée en ce qu'elle contient de plus de la cortisone (IV-cort).

4. Préparation en association selon la revendication 3, caractérisée en ce qu'elle contient les agents I-Chla, II-Gt, III-Ns et IV-cort à la proportion de (4 à 12) à (0,5 à 1,5) à (20 à 60) à (1,5 à 4,5).

5. Préparation en association selon les revendications 1 ou 2, caractérisée en ce qu'elle contient les agents actifs sous la forme d'un mélange dans une base de pommade.

6. Préparation en association selon la revendication 3, caractérisée en ce qu'elle contient, pour 100 g du mélange pour pommade, 100 à 300 mg de I-Chla, 10 à 35 g de II-Gt et 700 à 1.100 mg de III-Ns.

7. Préparation en association selon les revendications 3 ou 4, caractérisée en ce qu'elle contient les agents actifs sous la forme d'un mélange dans une base de pommade.

8. Préparation en association selon la revendication 3, caractérisée en ce qu'elle contient, pour 100 g du mélange pour pommade, 100 à 300 mg de I-Chla, 10 à 35 mg de II-Gt, 700 à 1.100 mg de III-Ns et 60 à 100 mg de IV-cort.

9. Préparation en association selon les revendications 1 à 4, caractérisée en ce qu'elle contient les agents actifs dans un liquide que les yeux peuvent supporter.

EP 0 432 638 B1

Figur 1

Figur 2

Figur 3

Figur 4

Figur 5A

Figur  5B

Figur 6A

Figur 6B

Figur 7A

EP 0 432 638 B1

Figur 7B